# DEMANDE DE BREVET EUROPEEN

(11) **EP 1 386 602 A1**
(43) Date de publication de la demande: **04.02.2004**
(21) Numéro de dépôt: 03291686.8
(22) Date de dépôt: 07.07.2003
(51) Int. Cl.: A61K 7/13, A61K 7/06

(54) **Procédé de préparation d'une composition tinctoriale pour la teinture de fibres kératiniques à partir de vapeur d'eau sous pression**

(30) Priorité: 26.07.2002 FR 0209515
(71) Demandeur: L'OREAL, 75008 Paris (FR)
(72) Inventeur: Grollier, Jean-François, 75006 Paris (FR); de la Mettrie, Roland, 78110 Le Vesinet (FR); Cottard, Francois, 92400 Courbevoie (FR)
(74) Mandataire: Fevrier, Murielle

(57) **Abrégé**

La présente invention a pour objet un procédé de préparation d'une composition tinctoriale destinée à la teinture des fibres kératiniques par percolation de vapeur d'eau sous pression au travers d'un composé pulvérulent adapté.

L'invention concerne aussi un dispositif de conditionnement d'un tel composé ainsi que le procédé de teinture mettant en oeuvre une telle composition.

## Description

La présente invention a pour objet un procédé de préparation d'une composition tinctoriale destinée à la teinture des fibres kératiniques, en particulier des fibres kératiniques humaines telles que les cheveux ainsi qu'un procédé de teinture des fibres kératiniques à partir de cette composition.

Il est connu de teindre les fibres kératiniques et en particulier les cheveux humains avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

Il est aussi connu de teindre les fibres kératiniques par une coloration directe. Le procédé classiquement utilisé en coloration directe consiste à appliquer sur les fibres kératiniques des colorants directs qui sont des molécules colorées et colorantes ayant une affinité pour les fibres, à laisser pauser, puis à rincer éventuellement les fibres. Les colorants directs peuvent notamment être choisis parmi les colorants du type nitrés benzéniques, anthraquinoniques, nitropyridiniques, des colorants du type azoïques, xanthéniques, acridiniques aziniques ou des colorants méthiniques. Ces colorants directs peuvent être de nature non ionique, anionique ou cationique.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration.

Les compositions tinctoriales sont en général des compositions aqueuses dans lesquelles les bases, coupleurs ou colorants directs doivent être solubilisés. Il arrive que le manque de solubilité de ces composés dans la composition tinctoriale diminue le pouvoir colorant de ces compositions. De plus, ce critère de solubilité réduit le nombre de bases coupleurs ou colorants directs qui peuvent être utilisés pour la teinture des fibres kératiniques.

Par ailleurs, lorsque ces bases coupleurs ou colorants sont obtenus à partir d'extraits naturels végétaux ou animaux, l'extraction de ces composés peut s'avérer difficile avec un rendement d'extraction faible.

On connaît déja des procédés de teinture de fibres kératiniques qui utilisent la vapeur d'eau. Ces procédés consistent à appliquer dans un premier temps une composition tinctoriale classique sur les cheveux et d'appliquer sur les cheveux enduit de cette composition de la vapeur d'eau pendant un temps très court. Ces procédés permettent d'améliorer en particulier l'uniformité de la coloration des racines aux pointes.

Le but de la présente invention est de fournir un nouveau procédé de préparation de composition tinctoriale simple à mettre en oeuvre et qui permet d'utiliser une grande variété de bases, coupleurs et colorants directs, en particulier les bases, coupleurs ou colorants directs faiblement solubles.

Ce but est atteint par la présente invention qui a pour objet un procédé de préparation d'une composition tinctoriale pour la teinture des fibres kératiniques caractérisé en ce qu'il comprend une étape de percolation de vapeur d'eau sous une pression d'au moins 3 bars au travers d'au moins un composé pulvérulent capable de générer une solution apte à teindre les fibres kératiniques.

Il est ainsi possible de disposer d'une plus grande variété de bases, coupleurs ou colorants directs et d'obtenir ainsi d'une gamme plus large de nuances pour la coloration des fibres kératiniques. De plus, ce procédé permet d'accroître le pouvoir colorant des compositions tinctoriales par une meilleure solubilisation des colorants ou précurseurs de colorants dans le milieu de teinture, et une meilleure diffusion dans le milieu à teindre.

Un autre objet de l'invention est un dispositif de conditionnement permettant de mettre en oeuvre le procédé de préparation de la présente invention.

L'invention a aussi pour objet un procédé de teinture mettant en oeuvre la composition tinctoriale obtenue par le procédé de l'invention.

Le procédé de la présente invention peut être mis en oeuvre à partir d'un dispositif classique permettant de générer de la vapeur d'eau sous pression. Un tel dispositif comprend une chambre résistant à la pression équipée d'un bloc thermique ainsi qu'un circuit d'acheminement de la vapeur produite vers le composé capable de générer une solution apte à teindre les fibres kératiniques. Selon un autre mode de réalisation, le dispositif comprend de plus un réservoir d'eau ainsi qu'une pompe permettant l'acheminement de l'eau vers la chambre.

Un dispositif particulièrement utile pour la mise en oeuvre du procédé de la présente invention est une machine à café du type "expresso". De telles machines sont bien connues de la technique. Par exemple, ces machines sont décrites dans les brevets AT 168405, US 2688911, DE 3243870 et IT 1265636.

La vapeur d'eau ainsi produite percole au travers du ou des composés pulvérulents capables de générer une solution apte à teindre les fibres kératiniques. On obtient ainsi une composition tinctoriale fluide qui peut être appliquée directement sur les fibres kératiniques ou qui peut être mélangée à un milieu approprié à la teinture des fibres kératiniques ou encore à toute composition tinctoriale classique connue de la technique.

Selon un mode de réalisation particulier de l'invention, l'étape de percolation est mise en oeuvre avec de la vapeur d'eau sous une pression d'au moins 4 bars, de préférence supérieure à 10 bars et tout particulièrement comprise entre 10 et 30 bars.

Les composés capables de générer une solution apte à teindre les fibres kératiniques peuvent être utilisés sous forme de poudre placée directement dans le dispositif générant de la vapeur d'eau sous pression dans un récipient destiné à cet usage. Ils peuvent être conditionnés dans un dispositif de conditionnement particulier comprenant un logement fermé délimité par au moins une paroi au moins en partie perméable à de la vapeur d'eau sous pression d'au moins 3 bars. De tels dispositifs sont par exemples décrits dans les demandes de brevets WO 00/56629, EP512470 ou dans le WO 99/03753.

Selon un mode de réalisation particulier, le logement est délimité par deux feuilles scellées. Selon un autre mode de réalisation, le logement est délimité par une barquette fermée par un couvercle.

Ces dispositifs peuvent être fabriqués à partir de matériaux tissés ou non tissés, en matière plastique ou végétales par exemple en cellulose, en métal tel que l'aluminium ou en composite. De tels dispositifs sont par exemple décrits dans les demandes de brevets WO 00/56629, EP512470 ou WO 99/03753.

Les composés pulvérulents capables de générer une solution apte à teindre les fibres kératiniques sont bien connus de la technique. De tels composés sont par exemple des bases d'oxydation, des coupleurs ou des colorants directs. Ces composés peuvent être des composés synthétisés de façon classique ou extraits d'espèces végétales ou animales, cet extrait étant obtenu à partir de tout l'organisme ou de partie de celui ci en utilisant par exemple les feuilles ou les racines. Les substances actives pouvant être extraites d'espèces végétales sont par exemple les quinones hydroxylées, les indigoïdes, les hydroxyflavones, les santalines A et B, l'isatine et ses dérivés, la brasiline et son dérivé hydroxylé. Les espèces végétales qui peuvent être utiles dans la présente invention sont par exemple le henné, l'indigotier, la matricaire, le rocou, l'orcanette. Les espèces animales qui peuvent être utilisées sont par exemple les cochenilles.

Les bases d'oxydation sont généralement choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la N,N-diéthyl 3-méthyl paraphénylènediamine, la N,N-bis-(β-hydroxyéthyl) paraphénylènediamine, la N,N-bis-(β-hydroxyéthyl) 2-méthyl paraphénylènediamine, la N,N-bis-(β-hydroxyéthyl) 2-chloro paraphénylènediamine, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N-(éthyl) N-(β-hyd roxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridin-3-ylamine; le (3-amino-pyrazolo[1,5-a]pyridin-7-yl)-méthanol; le 2-(3-amino-pyrazolo[1,5-a]pyridin-5-yl)-éthanol ; le 2-(3-amino-pyrazolo[1,5-a]pyridin-7-yl)-éthanol; le (3-amino-pyrazolo[1,5-a]pyridin-2-yl)-méthanol ; la 3,6-diamino-pyrazolo[1,5-a]pyridine ; la 3,4-diamino-pyrazolo[1,5-a]pyridine; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridin-5-ol ; 3-amino-pyrazolo[1,5-a]pyridin-4-ol ; la 3-amino-pyrazolo[1,5-a]pyridin-6-ol ; la 3-amino-pyrazolo[1,5-a]pyridin-7-ol ; ainsi que leurs sels d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Les coupleurs utiles dans le cadre de la présente invention sont par exemple les coupleurs métaphénylènediamines, méta-aminophénols, métadiphénols, naphtaléniques, hétérocycliques et leur sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que la soude, la potasse, l'ammoniaque, les aminés ou les alcanolamines.

Les colorants directs utiles selon l'invention sont choisis de préférence parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants:
- 1,4-diamino-2-nitrobenzène
- 1-amino-2 nitro-4-(β-hydroxyéthyl)aminobenzène
- 1-amino-2 nitro-4-bis(β-hydroxyéthyl)-aminobenzène
- 1,4-Bis(β-hydroxyéthylamino)-2-nitrobenzène
- 1 -β-hydroxyéthylamino-2-nitro-4-bis-(β-hydroxyéthylamino)-benzène
- 1-β-hydroxyéthylamino-2-nitro-4-aminobenzène
- 1 -β-hydroxyéthylamino-2-nitro-4-(éthyl)(β-hydroxyéthyl)-aminobenzène
- 1 -amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1 ,2-Bis-(β-hydroxyéthylamino)-4-nitrobenzène
- 1 -amino-2-tris-(hydroxyméthyl)-méthylamino-5-nitrobenzène
- 1 -Hydroxy-2-amino-5-nitrobenzène
- 1 -Hydroxy-2-amino-4-nitrobenzène
- 1 -Hydroxy-3-nitro-4-aminobenzène
- 1-Hydroxy-2-amino-4,6-dinitrobenzène
- 1 -β-hydroxyéthyloxy-2-β-hydroxyéthylamino-5-nitrobenzène
- 1 -Méthoxy-2-β-hydroxyéthylamino-5-nitrobenzène
- 1 -β-hydroxyéthyloxy-3-méthylamino-4-nitrobenzène
- 1 -β,γ-dihydroxypropyloxy-3-méthylamino-4-nitrobenzène
- 1 -β-hydroxyéthylamino-4-β,γ-dihydroxypropyloxy-2-nitrobenzène
- 1 -β,γ-dihydroxypropylamino-4-trifluorométhyl-2-nitrobenzène
- 1-β-hydroxyéthylamino-4-trifluorométhyl-2-nitrobenzène
- 1 -β-hydroxyéthylamino-3-méthyl-2-nitrobenzène
- 1 -β-aminoéthylamino-5-méthoxy-2-nitrobenzène
- 1 -Hydroxy-2-chloro-6-éthylamino-4-nitrobenzène
- 1 -Hydroxy-2-chloro-6-amino-4-nitrobenzène
- 1 -Hydroxy-6-bis-(β-hydroxyéthyl)-amino-3-nitrobenzène
- 1 -β-hydroxyéthylamino-2-nitrobenzène
- 1 -Hydroxy-4-β-hydroxyéthylamino-3-nitrobenzène.
Parmi les colorants directs azoïques, on peut citer les colorants azoïques cationiques décrits dans les demandes de brevets WO 95/15144, WO-95/01772 et EP-714954 dont le contenu fait partie intégrante de l'invention.
Parmi ces composés on peut tout particulièrement citer les colorants suivants: chlorure de 1,3-diméthyl-2-[[4-(diméthylamino)phényl]azo]-1 H-lmidazolium, chlorure de 1,3-diméthyl-2-[(4-aminophényl)azo]-1H-Imidazolium, méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3^{e} édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.

On peut aussi citer le 1-(4'-aminodiphénylazo)-2-mèthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3 , Disperse Red 11, Acid Blue 62, Disperse Blue 7, Basic Blue 22, Disperse Violet 15, Basic Blue 99, ainsi que les composés suivants : -1-N-méthylmorpholiniumpropylamino-4-hydroxyanthraquinone, 1-Aminopropylamino-4-méthylamino-anthraquinone, 1-Aminopropylamino-anthraquinone, 5-β-hydroxyéthyl-1,4-diaminoanthraquinone, 2-Aminoéthylamino-anthraquinone, 1,4-Bis-(β,γ-dihydroxypropylamino)-anthraquinone.

Parmi les colorants aziniques on peut citer les composés suivants : Basic Blue 17, Basic Red 2.

Parmi les colorants triarylméthaniques utilisables selon l'invention, on peut citer les composés suivants : Basic Green 1, Acid blue 9, Basic Violet 3, Basic Violet 14, Basic Blue 7, Acid Violet 49, Basic Blue 26, Acid Blue 7.

Parmi les colorants indoaminiques utilisables selon l'invention, on peut citer les composés suivants :
- 2-β-hydroxyéthlyamino-5-[bis-(4'-βhydroxyéthyl)amino]anilino-1,4-benzoquinone
- 2-β-hydroxyéthylamino-5-(2'-méthoxy-4'-amino)anilino-1 ,4-benzoquinone
- 3-N(2'-Chloro-4'-hydroxy)phényl-acétylamino-6-méthoxy-1 ,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1 ,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Dans le procédé de l'invention, les composés capables de générer une solution apte à teindre les fibres kératiniques peut être constitués d'une ou plusieurs bases d'oxydation, d'un ou plusieurs coupleurs, d'un ou plusieurs colorants directs, seuls ou en mélanges.

Le ou les composés utiles dans le procédé de la présente invention peuvent être mis en oeuvre en mélange avec un support de préférence pulvérulent. Un tel support peut être constitué de matière pulvérulente organique, minérale ou végétales servant de charges et d'adjuvants pulvérulents facilitant la mise en oeuvre et/ou la teinture. Les charges minérales peuvent être des argiles, des sels. Les charges organiques peuvent être des polymères tels que les sucres (mono-oligo ou polysaccharides). Les charges végétales peuvent être des poudres de racines, de feuilles, de protéines végétales. Les adjuvants pulvérulents peuvent être des agents basifiants ou acidifiants tels que la lysine ou l'acide citrique, des tensio-actifs tels que le laurylsulfate, des réducteurs tels que l'acide ascorbique ou les sulfites, des épaississants naturels ou de synthèse.

A partir du procédé de préparation de l'invention, on obtient une composition tinctoriale qui peut être mise en oeuvre soit directement soit après addition de différents composants.

La quantité de bases d'oxydation présentes dans la composition tinctoriale obtenue par le procédé de la présente invention est en général comprise entre 0,001 et 10 % en poids environ du poids total de la composition tinctoriale, de préférence entre 0,005 et 6 %.

La quantité de coupleurs présente dans la composition tinctoriale obtenue par le procédé de la présente invention est en général comprise entre 0,001 et 10 % en poids environ du poids total de la composition tinctoriale, de préférence entre 0,005 et 6 %.

La quantité de colorants directs présente dans la composition tinctoriale obtenue par le procédé de la présente invention est en général comprise entre 0,001 et 20% en poids environ du poids total de la composition prête à l'emploi et encore plus préférentiellement entre 0,005 et 10% en poids environ.

Lorsque la composition tinctoriale obtenue par le procédé de la présente invention est mélangée à un milieu approprié pour la teinture, un tel milieu est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono- , di- et triéthanolamines ainsi que leurs dérivés, les hydroxydes de sodium ou de potassium et les composés de formule (II) suivante : 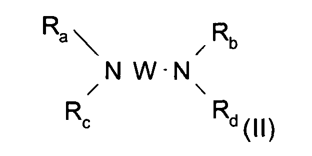 dans laquelle W est un reste propylène éventuellement substitué par un groupement hydroxyle ou un radical alkyle en C₁-C₄; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale finale peut se présenter sous des formes diverses, telles que sous forme de liquides, de crèmes, de gels ou sous toute autre forme appropriée pour réaliser une teinture des fibres kératiniques, et notamment des cheveux humains.

Lorsque le composé capable de générer une solution apte à colorer les fibres kératiniques comprend une base d'oxydation, la couleur est alors révélée à l'aide d'un agent oxydant.

Selon un mode de réalisation particulier, la composition est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

La couleur peut être révélée à pH acide, neutre ou alcalin.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

A titre d'exemple, une poudre de Henné est placée dans une machine expresso du commerce Magimix Nespresso ® dans le récipient prévu pour recevoir un composé pulvérulent. Ce composé pulvérulent est ensuite traversé par la vapeur d'eau produite par la machine. On obtient ainsi une composition tinctoriale prête à être appliquée sur les fibres kératiniques.

Une composition pulvérulente comprenant en mélange équimolaire du 4-amino-2-méthyl phénol et du 1-méthyl-2-hydroxy-4-aminobenzène est placée dans le récipient de même machine expresso prévu pour recevoir une matière pulvérulente. Ce mélange pulvérulent est traversé par de la vapeur d'eau produite par la machine. La composition tinctoriale ainsi obtenue est mise en contact avec un agent oxydant tel que le peroxyde d'hydrogène avant application sur les fibres kératiniques.

## Revendications

1. Procédé de préparation d'une composition tinctoriale pour la teinture des fibres kératiniques **caractérisé en ce qu'**il comprend une étape de percolation de vapeur d'eau sous une pression d'au moins 3 bars au travers d'au moins un composé pulvérulent capable de générer une solution apte à teindre les fibres kératiniques.

2. Procédé selon la revendication 1 dans lequel le composé capable de générer une solution apte à teindre les fibres kératiniques est choisie parmi les bases d'oxydation, les coupleurs ou les colorants directs.

3. Procédé selon la revendication 1 ou 2 dans lequel le composé capable de générer une solution apte à teindre les fibres kératiniques est une base d'oxydation choisie parmi les paraphénylènediamines, les bis-phénylalkylènediamines, les para-aminophénols, les ortho-aminophénols, les bases hétérocycliques et leurs sels d'addition.

4. Procédé selon l'une quelconque des revendications 1 à 3 dans lequel le composé capable de générer une solution apte à teindre les fibres kératiniques est un coupleur choisi parmi les métaphénylènediamines, les méta-aminophénols, les métadiphénols, les coupleurs naphtaléniques, les coupleurs hétérocycliques et leurs sels d'addition.

5. Procédé selon l'une quelconque des revenications 1 à 3 dans lequel le composé capable de générer une solution apte à teindre les fibres kératiniques est un colorant direct choisi parmi les colorants nitrés de la série benzénique, les colorants directs azoïques, les colorants directs méthiniques, ces colorants directs pouvant être de nature non ionique, anionique ou cationique.

6. Procédé selon l'une quelconque des revendications 1 à 5 dans lequel le composé capable de générer une solution apte à teindre les fibres kératiniques est issu d'une espèce végétale ou animale.

7. Procédé selon l'une quelconque des revendications précédentes dans lequel l'étape de percolation est mise en oeuvre avec de la vapeur d'eau sous une pression supérieure à 10 bars.

8. Procédé de teinture des fibres kératiniques **caractérisé en ce qu'**on prépare une composition tinctoriale selon le procédé défini selon l'une quelconque des revendications 1 à 7 et qu'on applique cette composition sur les fibres kératiniques pendant un temps suffisant pour développer la coloration désirée.

9. Dispositif de conditionnement d'un composé capable de générer une solution apte à teindre les fibres kératiniques comprenant un logement fermé délimité par au moins une paroi au moins en partie perméable à de la vapeur d'eau sous pression d'au moins 3 bars, le logement contenant au moins un composé pulvérulent capable de générer une solution apte à teindre les fibres kératiniques.

10. Dispositif selon la revendication 9 dans lequel le logement est délimité par deux feuilles scellées.

11. Dispositif selon la revendication 9 dans lequel le logement est délimité par une barquette fermée par un couvercle.
